# EUROPÄISCHE PATENTSCHRIFT

(11) **EP 1 633 374 B1**
(45) Veröffentlichungstag und Bekanntmachung des Hinweises auf die Patenterteilung: **15.08.2007**
(21) Anmeldenummer: 04734822.2
(22) Anmeldetag: 26.05.2004
(51) Int. Cl.: A61K 31/78, A61K 31/19

(54) **ZUSAMMENSETZUNG AUS ALKANDICARBONSÄUREN UND EINEM PHARMAZEUTISCHEN WIRKSTOFF**
COMPOSITION CONSISTING OF ALKANE DICARBOXYLIC ACIDS AND A PHARMACEUTICALLY ACTIVE INGREDIENT
COMPOSITION A BASE D'ACIDES ALKYLDICARBOXYLIQUES ET D'UN PRINCIPE ACTIF PHARMACEUTIQUE

(30) Priorität: 10.06.2003 DE 10326722
(43) Veröffentlichungstag der Anmeldung: 15.03.2006
(73) Patentinhaber: Intendis GmbH, 10589 Berlin (DE)
(72) Erfinder: JOHANNSEN, Sönke, 10437 Berlin (DE); ZOLLNER, Thomas, 12161 Berlin (DE)
(74) Vertreter: Seuss, Thomas
(86) Internationale Anmeldenummer: PCT/EP2004/005668
(87) Internationale Veröffentlichungsnummer: WO 2004/108143

(56) Entgegenhaltungen:
- WO-A-89/06537
- US-A- 4 713 394
- MADDIN S: "A COMPARISON OF TOPICAL AZELAIC ACID 20% CREAM AND TOPICAL METRONIDAZOLE 0.75% CREAM IN THE TREATMENT OF PATIENTS WITH PAPULOPUSTULAR ROSACEA" JOURNAL OF THE AMERICAN ACADEMY OF DERMATOLOGY, C.V. MOSBY, ST. LOUIS, MO, US, Bd. 40, Nr. 6, PART 1, Juni 1999 (1999-06), Seiten 961-965, XP008016063 ISSN: 0190-9622
- BLEICHER P A ET AL: "TOPICAL METRONIDAZOLE THERAPY FOR ROSACEA" ARCHIVES OF DERMATOLOGY, XX, XX, Bd. 123, Nr. 5, Mai 1987 (1987-05), Seiten 609-614, XP008016055 ISSN: 0003-987X

## Beschreibung

Die Erfindung betrifft eine Zusammensetzung aus Alkandicarbonsäuren und einem weiteren pharmazeutischen Wirkstoff, insbesondere als pharmazeutische Zusammensetzung zur Behandlung von Rosacea. Weiterhin umfasst die Erfindung die Verwendung der Zusammensetzung zur Herstellung eines Medikaments zur Behandlung von Rosacea.

### Stand der Technik

### Zusammensetzung mit Azelainsäure

Die deutsche Patentanmeldung DE 28 17 133 (Anmeldetag 19.4.1978) von Nazzaro-Porro beschreibt Alpha,omega-n-alkandicarboxyl Säuren mit 7 bis 13 Kohlenstoffatomen und deren Ester, die von Hautenzymen spaltbar sind. Bevorzugt sind Pimelinsäure, Suberinsäure, Azelainsäure, Sebacinsäure, 1,9-Nonandicarbonsäure, 1,10-Decandicarbonsäure und 1,11-Undecandicarbonsäure. Azelainsäure ist am meisten bevorzugt. Die Dicarbonsäuren werden zur Behandlung von hyperpigmentären Dermatosen oder von Hauthyperpigmentationen eingesetzt.

Das europäische Patent EP 0 305 407 (Anmeldetag 07.07.1987) von Nazzaro-Porro stellt die Verwendung von Dicarbonsäuren mit 7 bis 13 Kohlenstoffatomen, insbesondere Azelainsäure, zur Behandlung von Rosacea unter Schutz.

In der EP 0 336 880 A2, die am 29.3.1998 angemeldet worden ist, wird eine pharmazeutische Zusammensetzung beschrieben, die besteht aus Azelainsäure in einer Konzentration von 20 Gewichtsprozenten und aus pharmazeutischen Zusatzstoffen und Trägern wie Triacylglyceriden und Diacylglyceriden, Propylenglykol, Polysorbat, zum Beispiel Polyethylen(20)sorbitanmonooleat, und Wasser und Salzen. Diese topisch zu verabreichende Zusammensetzung wird zur Behandlung von verschiedenen altersbedingten Veränderungen der Gesichtshaut eingesetzt. Die Zusammensetzung liegt als Creme vor. Bekannt ist weiterhin, die Azelainsäure auch gegen entzündliche Dermatosen, wie zum Beispiel Akne und Rosacea, einzusetzen.

In der Roten Liste von 1996 (ISBN 3 - 87193 - 167 -5) wird unter der Nummer 32 282 eine pharmazeutische Zusammensetzung mit dem Namen Skinoren beschrieben, die besteht aus Azelainsäure in einer Konzentration von 20 Gewichtsprozenten und aus pharmazeutischen Trägerstoffen und Zusatzstoffen, wie Triacylglyceriden und Diacylglyceriden, Propylenglycol, Polysorbat, zum Beispiel Macrogolstrearat 1000 und Wasser und Salzen. Diese topisch zu verabreichende Zusammensetzung wird zur Behandlung von Akne eingesetzt. Die Zusammensetzung liegt als Creme vor.

In dem europäischen Patent EP 1 032 379 B1 wird ein Hydrogel mit Azelainsäure beschrieben, bei der die folgenden Zusatzstoffe eingesetzt werden: Triacylglyceride, Propylenglycol, Polyacrylsäure, Sojalecithin und Polysorbate in einer wässrigen Phase, umfassend Wasser und Salze. Die Zusammensetzung wird zur Behandlung von Rosacea, Altershaut, Melasma, Akne und / oder Hautirritationen eingesetzt.

### Zusammensetzung mit Metronidazol

In der US-Patentschrift US 2,944,061 (Publikationstag 5. Juli 1969) von R.M. Jacob et al. werden neue Imidazolderivate beschrieben, welche chemotherapeutische Wirkung aufweisen, insbesondere zur Behandlung von Infektionen mit Protozoen, wie pathogene Amöben oder Trichomonaden.

Das US-Patent US 4,837,378 (Publikationstag am 6. Juni 1989) von R.J. Borgman befasst sich mit einer dermatologischen Zusammensetzung zur topischen Verabreichung in Gelform, wobei der pharmazeutische Wirkstoff Metronidazol ist. Als Gelbildner werden Polymere mit freien Carboxylgruppen, wie Polyacrylsäure mit einem Molekulargewicht von 1·10⁶ bis 4·10⁶ Dalton eingesetzt.

Mit der dermatologischen Zusammensetzung werden Rosacea und Akne vulgaris behandelt.

### Aufgabe und Lösung

Es stellt sich die Aufgabe, eine Zusammensetzung mit einer Dicarbonsäure als therapeutischen Wirkstoff anzubieten, wobei ein weiterer pharmazeutischer Wirkstoff mitumfasst ist. Dabei soll die Zusammensetzung der beiden pharmazeutischen Wirkstoffe deutlich anti - inflammatorischer wirken als jede einzelne Substanz. Insbesondere soll die anti - inflammatorsche Zusammensetzung zur Behandlung von Psoriasis, atopischer Dermatitis, Akne vulgaris und Rosacea eingesetzt werden.

Die Aufgabe wird durch eine Zusammensetzung gelöst, die die beiden folgenden Stoffe umfasst:
(i) mindestens eine Alpha,omega-n-alkandicarboxyl Säuren mit 7 bis 13 Kohlenstoffatomen oder deren Ester und
(ii) mindestens ein Imidazolderivat mit der allgemeinen Formel 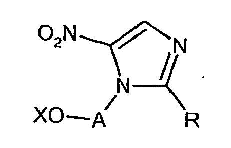
wobei
- R: ein Wasserstoffatom oder eine Alkylgruppe ist, die bis zu fünf Kohlenstoffatome enthält, wobei Methyl- und Ethylgruppen bevorzugt sind,
- A: eine gesättigte lineare Kette einer aliphatischen Hydrocarbongruppe ist, die zwei bis fünf Kohlenstoffatome enthält,
- X: ein Wasserstoffatom oder ein Akylrest einer monocarboxylischen oder dicarboxylischen aliphatischen oder aromatischen Säure ist.

Bevorzugt sind Alpha,omega-n-alkandicarboxyl Säuren oder deren Ester die aus der folgenden Gruppe ausgewählt sind: Pimelinsäure, Suberinsäure, Azelainsäure, Sebacinsäure, 1,9-Nonandicarbonsäure, 1,10-Decandicarbonsäure und 1,11-Undecandicarbonsäure. Mehr bevorzugt ist Azelainsäure oder deren Ester und am meisten bevorzugt ist Azelainsäure.

Als Estergruppen können Alkylgruppen von 1 bis 5 Kohlenstoffatomen unabhängig voneinander an der alpha und der omega Position eingesetzt werden. Wesentlich ist, dass die Ester von den Hautenzymen spaltbar sind. Alkylgruppen können Methyl, Ethyl, Propyl, Butyl oder Pentyl in gerader oder verzweigter Form sein.

Bevorzugt ist ein Imidazolderivat aus der Gruppe:
1-(2-hydroxyethyl)-2-methyl-5-nitroimidazol;
1-(2-benzoyloxyethyl)-2-methyl-5-nitroimidazol;
1-(2-succinoyloxyethyl)-2-methyl-5-nitroimidazol; und
1-(2-cinnamoyloxyethyl)-2-ethyl-5-nitroimidazol.

Am meisten bevorzugt ist Metronidazol = 1-(2-hydroxyethyl)-2-methyl-5-nitroimidazol.

### Vorteile

Bei der Behandlung der entzündlichen Form der Rosacea haben sich u.a. die zugelassenen Präparate Metronidazol (0.5 - 1.0 % in verschiedenen Vehikeln) und Azelainsäure (20 % in verschiedenen Vehikeln) bewährt (Rebora, A. (2002) The management of rosacea. Am. J. Clin. Dermatol. Vol: 3(7), pp 489-96). Die Wirkung einer kombinatorischen Behandlung von Azelainsäure und Metronidazol wurde bisher nicht in vivo untersucht, es liegen lediglich Daten zum Vergleich der Wirksamkeit der Einzelsubstanzen bei Rosacea vor (Maddin (1999) Journal of the A merican Academiy of Dermatology, Vol 40(69 pp 961 - 965). Außerdem sind keine Untersuchungsergebnisse publiziert, die eine additive oder synergistische Wirkung einer Kombinationstherapie erwarten ließen.

Erstaunlicher Weise wurde gefunden, dass eine Kombinationstherapie von Metronidazol (0.75%) und Azelainsäure (20%) in Ethanol/lsopropylmyristat in einem Entzündungsmodell im Vergleich zum Vehikel signifikant therapeutisch effektiv sind, wohingegen die beiden Einzelsubstanzen Metronidazol (0.75%) bzw. Azelainsäure (20%) keine so hohen, therapeutischen Effekte im Vergleich zum Vehikel zeigen. Die Wirksamkeit beinhaltete u.a. die Einwanderung von Leukozyten gemessen als Peroxidase-Aktivität in der Haut sowie die Einwanderung von neutrophilen Granulozyten gemessen als Elastase-Aktivität in der Haut. Die Einwanderung dieser Zellpopulation in die Haut und Freisetzung proinflammatorischer Substanzen ist in die Pathogenese einer Reihe entzündlicher Erkrankungen involviert. Diese Erkrankungen beinhalten u.a. Rosacea, Akne, allergische und irritative Kontaktdermatitiden, atopische Dermatitis und Psoriasis (Braun - Falco, O.,G. Plewig et al. (1995) Dermatologie und Venerologie, Heidelberg, Springer).

Die Induktion einer entzündlichen Reaktion (irritative Kontaktdermatitis) ist ein weit verbreitetes Modell zur pharmakologischen Evaluierung des anti - inflammatorischen Potentials von topisch oder systemisch applizierten Testsubstanzen. (Trancik and Lowe (1985) Evaluation of topical nonsteroidal anti - inflammatory agents, Models in Dermatology, H. Maibach and N.J. Lowe, Basel Karger: 35 - 42) Es hat sich unter anderem bei der Testung von nicht - steroidalen Antiphlogistika sowie bei Glukokortikosteroiden bewährt und gilt als der am besten reproduzierbare in vivo Testversuch für die Evaluierung topischer anti - inflammatorischer Substanzen (Lorenzetti (1975) Animal to Clinical Correlation of Topical Anti - Inflammatory Efficacy, Animal models in dermatology. H. Maibach, Basel Karger: 212 - 224)

### Weitere Ausführungsformen

Mehr bevorzugt ist eine erfindungsgemäße Zusammensetzung, die eine pharmazeutische Zusammensetzung mit pharmazeutischen Trägerstoffen und Hilfsstoffen umfasst, die in Remington's Pharmaceutical Science, 15th ed. Mack Publishing Company, Easton Pennsylvania (1980) beschrieben sind.

### Weitere Aufgabe und Lösung

Weiterhin soll die Bioverfügbarkeit der beiden Wirkstoffe gesteigert werden.

Die pharmazeutischen Zusammensetzung aus mindestens
einer Alpha,omega-n-alkandicarboxyl Säure mit 7 bis 13 Kohlenstoffatomen oder deren Ester und
mindestens einem Imidazolderivat mit der zuvor genannten allgemeinen Formel
mit den folgenden pharmazeutischen Trägerstoffen:
Triacylglyceride, Propylenglycol, Polysorbate, Polyacrylsäure, Sojalecithin in einer wässrigen Phase, umfassend Wasser und Salze umfasst, wobei die Zusammensetzung ein Hydrogel ist.

### Vorteile

Die Zusammensetzung gemäß der Erfindung weist den Vorteil auf, dass sie eine höhere Menge an pharmazeutischem Wirkstoff in lebende Hautschichten und /oder Hautanhangsorganen gelangen lässt. Diese Verfügbarkeit ist in einem FRANZ - Durchfluss - Diffusionszell - Test nachweisbar.

### Weitere Ausgestaltung der Zusammensetzung

Vorteilhaft ist eine Zusammensetzung, welche topisch verabreichbar ist.

Wesentlich ist die Gegenwart von Polyacrylsäure. Sie ist für das Entstehen des Hydrogels entscheidend. In dem Gel ist als Lecithin das Sojalecithin bevorzugt. Das Lecithin oder Sojalecithin ist in einer Konzentration von mindestens 3 Gewichtsprozenten vorteilhaft. Mehr bevorzugt eine Konzentration von mindestens 1,5 Gewichtsprozenten und am meisten bevorzugt eine Konzentration von mindestens 1 Gewichtsprozent.

### Vorteile

Vorteilhafter Weise besitzt die erfindungsgemäße Zusammensetzung eine hohe Penetration in lebende Hautschichten und / oder Hautanhangsorganen.

### Bevorzugte Ausführungsformen

Bevorzugt ist eine erfindungsgemäße Zusammensetzung, bei der die einzelnen Parameter unabhängig voneinander die folgenden Konzentrationen aufweisen können:
Polyacrylsäure in einer Konzentration von 0,5 bis 2 Gewichtsprozenten,
Triacylglyceride in einer Konzentration von 0,5 bis 5 Gewichtsprozenten,
Propylenglycol in einer Konzentration von 5 bis 15 Gewichtsprozenten und
Polysorbate in einer Konzentration von 0,5 bis 3 Gewichtsprozenten.

Die Bestandteile sind selbstverständlich so aufeinander abzustimmen, dass eine Summe von 100 % erzielt wird.

Am meisten bevorzugt ist eine Zusammensetzung, bei der die einzelnen Parameter unabhängig voneinander die folgenden Konzentrationen aufweisen können:
Polyacrylsäure in einer Konzentration von 1 ± 0,25 Gewichtsprozenten,
Triacylglyceride in einer Konzentration von 2 ± 1 Gewichtsprozenten,
Propylenglycol in einer Konzentration von 10 ± 2 Gewichtsprozenten und
Polysorbate in einer Konzentration von 2 ± 0,5 Gewichtsprozenten.

Die Bestandteile sind selbstverständlich so aufeinander abzustimmen, dass eine Summe von 100 % erzielt wird.

### Definitionen

Azelainsäure und deren Herstellung ist in dem deutschen Patent DE 28 17 133.7 beschrieben. Vergleiche auch Römpp, Chemie Lexikon, Herausgeb. Jürgen FALBE und Manfred REGNITZ, 1989, 9. Auflage, Seite 323, Thieme Verlag Stuttgart, ISBN 3-13-734609-6.

Metronidazol und dessen Herstellung ist in der US-Patentschrift US 2,944,061 (Publikationstag 5. Juli 1969) von R.M. Jacob et al. beschrieben. Dort werden neue Imidazolderivate und insbesondere Metronidazol charakterisiert. Das US-Patent US 4,837,378 (Publikationstag am 6. Juni 1989) von R.J. Borgman befasst sich mit einer topischen Verabreichung von Metronidazol. Mit der dermatologischen Zusammensetzung werden Rosacea und Akne vulgaris behandelt.

Polyacrylsäure stellt ein anionenaktives Polymerisat aus Acrylsäure dar, das in Wasser nur zum Teil löslich ist. Die einprozentige wässrige Suspension besitzt einen pH-Wert von 3 und annähernd gleiche Viskosität wie Wasser. Erst beim Neutralisieren mit anorganischen oder organischen Basen kommt es zur Gelbildung und zum Entstehen hochviskoser Produkte. Rudolf VOIGT und Manfred BORNSCHEIN, 1979, Lehrbuch der pharmazeutischen Technologie, Seite 314, VEB Verlag Volk und Gesundheit Berlin. Vergleiche auch Römpp, Chemie Lexikon, Herausgeb. Jürgen FALBE und Manfred REGNITZ, 1992, 9. Auflage, Seite 3508, Thieme Verlag Stuttgart, ISBN 3-13-735009-3.

"Triglycerid" ist eine Bezeichnung für Ester des Glycerins, dessen drei Hydroxy - Gruppen durch Karbonsäuren verestert sind. Die natürlich vorkommenden Fette und fetten Öle sind Triglyceride ("Neutralfette"), die in der Regel verschiedene Fettsäuren im gleichen Glycerin-Molekül enthalten. J. Am.- Oil. Chem Soc. Vol 62, Seite 730, (1985); und Parfüm. Kosmet. Vol 58, Seite 353, (1977); und Römpp, Chemie Lexikon, Herausgeb. Jürgen FALBE und Manfred REGNITZ, 1990, 9. Auflage, Seiten 1339 - 1342, Thieme Verlag Stuttgart, ISBN 3-13-734709-2.

Propylenglycol ist in H .P. F IEDLER: Lexikon der Hilfsstoffe, 4. Auflage, 1996, ISBN 3-87193-173 auf den Seiten 1278 bis 1282 beschrieben.

Polysorbate sind in H.P. FIEDLER: Lexikon der Hilfsstoffe, 4. Auflage, 1996, ISBN 3-87193-173 auf den Seiten 1251 bis 1252 beschrieben.

Lecithine werden durch Extrahieren aus biologischem Material gewonnen. So umfasst eine Lecithin - Fraktion aus Sojabohnen (dem gebräuchlichsten Rohstoff) z.B. Palmitinsäure, Stearinsäure, Palmitoleinsäure, Oleinsäure, Linolsäure u. Linolensäure. Normalerweise ist die gesättigte Fettsäure mit der primären, die ungesättigte mit der sekundären Hydroxy-Gruppe des Glyzerins verestert. Le-cithine sind Bestandteile der Zellmembranen aller Lebewesen. In Wasser quellen Lecithine zunächst wie lyophile Kolloide auf. Später stellen sie durchsichtige, kolloidale Lösungen dar. Je nach Wassergehalt bilden sie unterschiedliche Texturen, wobei die Lamellen aus Lipid - Doppelschichten gebildet werden. Bei höherem Wassergehalt entstehen Liposomen. Lit.: Pardun, Die Pflanzenlecithine, Augsburg: Verl. für Chem. lnd. (Ziolkowsky KG) 1988. Weitere Lecithine und deren Wirkung sind beschrieben in J. GAREISS et al. 1994, Parfümerie und Kosmetik, Vol. 10/94, Seiten 652 - 659, Hüthing GmbH Heidelberg.

Ein Gel zeichnet sich durch die folgenden Eigenschaften aus: Es handelt sich um ein formbeständiges, leicht deformierbares, an Flüssigkeit und gegebenenfalls Gasen reiches, disperses System aus mindestens zwei Komponenten. Römpp, Chemie Lexikon, Herausgeb. Jürgen FALBE und Manfred REGNITZ, 1990, 9. Auflage, Seite 1511, Thieme Verlag Stuttgart, ISBN 3-13-734709-2; und Pharm. Unserer Zeit Vol. 8, Seiten 179 bis 188, (1979); und Parfüm. Kosmet., Vol. 58, Seiten 251 bis 253 (1977)

Konservierungsmittel können in der wässrigen Phase enthalten sein. Zu den Konservierungsmittel gehört zum Beispiel Benzoesäure. Aufgrund ihrer antimikrobiellen Eigenschaft ist Benzoesäure als Konservierungsmittel besonders geeignet.

### Eigenschaften bei der Verwendung als Medikament

### Pharmazeutischer Wirkstoff:

Die Zusammensetzung der Erfindung zeigt pharmakologische Wirkung in einem etablierten Testsystem zur Evaluierung topischer anti - inflammatorischer Substanzen (O.J. Lorenzetti (1975) Animal to Clincal Correlation of Topical Anti - Inflammatory Efficacy. Animal models in dermatology, H. Maibach, Basel, Karger, pp 212 - 225). Daher ist die Zusammensetzung als therapeutischer Wirkstoff oder als Medikament einsetzbar.

Die Zusammensetzung der Erfindung eignet sich zur Behandlung verschiedener Indikationen. Bevorzugt ist eine Zusammensetzung der Erfindung als therapeutischer Wirkstoff zur Behandlung von Psoriasis, atopischer Dermatitis, Akne vulgaris und / oder Rosacea. Weiterhin umfasst die Erfindung die Verwendung einer erfindungsgemäßen Zusammensetzung zur Herstellung eines Medikaments zur Behandlung von Psoriasis, atopischer Dermatitis, Akne vulgaris und / oder Rosacea.

Mehr bevorzugt ist eine Zusammensetzung der Erfindung als therapeutischer Wirkstoff zur Behandlung von Rosacea oder Psoriasis zusammen mit mindestens einem physiologisch verträglichen, pharmakologischen Hilfsstoff oder Trägerstoff.
(i) Die Erfindung liefert weiterhin
   (α) die Verwendung der pharmazeutischen Zusammensetzung der Erfindung zur Herstellung eines Medikaments zur Behandlung von Psoriasis, atopischer Dermatitis, Akne vulgaris und / oder Rosacea;
   (β) ein Verfahren zur Behandlung von Psoriasis, atopischer Dermatitis, Akne vulgaris und / oder Rosacea, welches Verfahren eine Verabreichung einer pharmazeutischen Zusammensetzung gemäß der Erfindung umfasst, wobei die Menge die Krankheit unterdrückt, und wobei die pharmazeutische Zusammensetzung einem Patienten gegeben wird, der ein solches Medikament benötigt;
   (γ) eine pharmazeutische Zusammensetzung zur Behandlung von Psoriasis, atopischer Dermatitis, Akne vulgaris und / oder Rosacea,
      welche Behandlung eine pharmazeutische Zusammensetzung der Erfindung und wenigstens einen pharmazeutisch verträglichen Träger und Zusatz umfasst.

Für diese therapeutische Wirkungen ist die geeignete Dosis unterschiedlich und hängt beispielsweise von der Konzentration der Einzelelemente in der pharmazeutischen Zusammensetzung, dem Wirt, der Art der Verabreichung und der Art und der Schwere der zu behandelnden Zustände ab.

Bevorzugte Konzentrationen an Alpha,omega-n-alkandicarboxyl Säuren mit 7 bis 13 Kohlenstoffatomen, insbesondere Azelainsäure, liegen in dem Bereich 2 bis 40 % (Gewichtsprozente), mehr bevorzugt 5 bis 20 %, noch mehr bevorzugt 8 bis 17 % und am meisten bevorzugt 10 - 15%. Bevorzugt ist eine Konzentration an lmidazolderivat und deren Ester, insbesondere Metronidazol von mindestens 0,1 % bis 3 % (Gewichtsprozent), mehr bevorzugt von 0,25 % bis 1 %, am meisten bevorzugt 0,7 % bis 0,8 %.

Die Behandlung umfasst prophylaktische und therapeutische Maßnahmen. Die Bestandteile der Zusammensetzung können zeitgleich und / oder räumlich in einer Dosierung verabreicht werden, weiterhin können sie körperlich und / oder zeitlich getrennt voneinander appliziert werden.

### Pharmazeutische Zubereitungen:

Die pharmazeutische Zusammensetzung der Erfindung kann mit den in der galenischen Pharmazie üblichen Zusätzen und/oder Trägersubstanzen nach an sich bekannten Methoden zu den üblichen topischen Applikationsformen verarbeitet werden. Bevorzugt ist eine erfindungsgemäße Zusammensetzung zusammen mit mindestens einem physiologisch verträglichen, pharmakologischen Hilfsstoff oder Trägerstoff. Pharmakologische Hilfsstoffe und Trägerstoffe sind in Remington's Pharmaceutical Science, 15th ed. Mack Publishing Company, Easton Pennsylvania (1980) beschrieben.

Die pharmazeutischen Zusammensetzung der Erfindung kann bei topischer Behandlung auf jedem üblichen Weg verabreicht werden, indem die Wirkstoffe mit geeigneten Zusätzen in die gewünschte Applikationsform, wie zum Beispiel Lösungen, Lotionen, Creme, Salben, Pasten oder Gel überführt. Das Gel ist bevorzugt. Die Lotionen, Cremes, Salben und Gele können in konventioneller Weise unter Verwendung konventioneller Emulgatoren hergestellt werden. (Kirk Othmer: Enzyclopedia of Chemical Technology, 3. Auflage, 1979, John Wiley & Sons, New York, Vol. 8, Seiten 900 - 930, und Dr. Otto-Albrecht Neumüller: Römpps Chemie Lexikon, 7. Auflage, 1973 Franckh' sche Verlagshandlung Stuttgart, Seiten 1009 - 1013).

Dem Arzneimittel können keratolytische Mittel, wie zum Beispiel Salizylsäure, Vitamin-A-Säure, Resorcin, Phenol, Cresol und dergleichen, zugesetzt werden.

Zu den physiologisch verträglichen Salzen zählen Alkalimetallsalze, wie Natrium- und Kaliumsalze, ferner Salze mit basischen Aminoverbindungen und organischen Aminen, wie zum Beispiel Arginin, Lysin oder N-Methylglutamin.

Als hydrophile und / oder lipophile Zusätze können Feuchthaltefaktoren (Hydrokomplexe), wie zum Beispiel Propylenglykol, Glycerin, Polyethylenglykole oder Aminosäuregemische, Puroba-Oil (=Jojoba-Öl), Vitamine (vorzugsweise Vitamin A und E), Vitalkomplexe (wie zum Beispiel Plazentaextrakte), Enzyme, Kräuterauszüge (wie zum Beispiel Hammamelis Extrakt oder Kamillenextrakt) oder Proteine (wie zum Beispiel Collagen) eingesetzt werden. Als ölige Phase oder als Fettphase in der Öl / Wasser - Emulsion eignen sich Kohlenwasserstoffe, wie zum Beispiel Sqalen, Vaseline, Paraffine oder Stearin, oder Wachse, wie zum Beispiel Bienenwachs. Geeignete Öl / Wasser - Emulgatoren sind beispielsweise Stearylalkohol, Polyoxyäthylenstearate (wie zum Beispiel MYRJ®, Komplexemulgatoren (wie zum Beispiel Amphoterin® und Sorbitanfettsäureester (wie zum Beispiel Tween 80®) oder Carboxyvinylpolymerisate (wie zum Beispiel Carbopol^{®}. Die wässrige Phase kann zusätzlich noch Puffersubstanzen, wie zum Beispiel das Dinatriumsalz der Äthylendiamin-N,N,N',N'-tetraessigsäure und Konservierungsmittel, wie Benzoesäure, Chlorquinaldol, Parabene oder Benzalkoniumchlorid enthalten.

### Indikationen:

### Rosacea:

Rosacea ist eine initial chronisch hyperämische (Rosacea 1), später chronisch entzündliche (Rosacea II und III) Krankheit des Gesicht. Sie ist auf erhöhte vaskuläre Reaktionen sowie entzündliche Prozesse mit Einwanderung von Leukozyten und Freisetzung entzündlicher Mediatoren zurückzuführen. Sie zeichnet sich aus durch ein persistierendes Erythem mit akuten Entzündungsphasen unter Ausbildung von Ödemen, Papeln und Pusteln. Sie tritt gewöhnlich mit einem Alter von über 20 Jahren auf. Azelainsäure, Antibiotika und Kortikosteroide werden zur Behandlung benutzt.

Die erfolgreiche Behandlung der Rosacea ist schwierig. Die erfindungsgemäße Zusammensetzung ist geeignet, einen günstigen Effekt sowohl auf die entzündliche Komponente (Papeln und Pusteln) als auch auf die vaskulare Komponente der Rosacea (Erythem und Teleangiektasien) auszuüben.

### Akne:

Die erfindungsgemäße Zusammensetzung zeigt gute Wirkung bei der Behandlung von Akne. Akne ist eine Anomalie der Talgdrüsen mit entzündlichen Pa-peln, Pusteln und Zysten und mit nicht - entzündlichen Mitessern. Sie befällt in den meisten Fällen Jugendliche und junge Erwachsene.

Die erfindungsgemäße Zusammensetzung ist geeignet, einen günstigen Effekt auf Akne auszuüben. Der günstige therapeutische Effekt der erfindungsgemäßen Zusammensetzung zeigt sich in deutlichem Rückgang der Papeln, Pusteln und Zysten.

### Psoriasis:

Psoriasis ist eine gewöhnliche, chronische, wuchernde Hautkrankheit, bei der die kerationozytische epidermale Durchgangszeit deutlich vergrößert ist. Die Läsionen sind scharf abgegrenzte, dicke, erythematose Flecken mit üppiger weißer Schuppung. Üblicher Weise werden die Ellbogen, die Knie, die Kopfhaut, die Genitalien und die Gesäßfalten befallen.

Die erfindungsgemäße Zusammensetzung ist geeignet, einen günstigen Effekt auf die Läsionen auszuüben.

### Beispiel

Die Induktion einer entzündlichen Reaktion (irritative Kontaktdermatitis) ist ein weit verbreitetes Modell zur pharmakologischen Evaluierung des anti - inflammatorischen Potentials von topisch oder systemisch applizierten Testsubstanzen (Trancik and Lowe (1985) Evaluation of topical nonsteroidal anti - inflammatory agents, Models in Dermatology, H. Maibach and N.J. Lowe. Basel, Karger: 35 - 42). Es hat sich u.a. bei der Testung von nicht - steroidalen Antiphlogistika sowie bei Glukokortikosteroiden bewährt und gilt als der am besten reproduzierbare in vivo Versuch für die Evaluierung topischer anti - inflammatorischer Substanzen (Lorenzetti (1975) Animal to Clinical correlation of Topical Anti - inflammatory Efficacy. Animal models in dermatology. H. Maibach. Basel Karger: 212 - 225).

Die Applikation des Phorbolesters Crotonöl auf Mäuseohren führt zu einer akuten, entzündlichen Reaktion mit Ödem und Einwanderung von vorwiegend polymorphkernigen Granulozyten, die binnen 24h ihr Maximum erreicht. Testsubstanzen können hierbei entweder präventiv (d.h. vor Applikation des entzündlichen Reizes) oder simultan mit Crotonöl appliziert werden. Die Crotonöl - induzierte Entzündung am Mäuseohr ist nicht nur ein Modell der irritativen und/oder allergischen Kontaktdermatitis, sondern auch anderer Hauterkrankungen, die mit einer starken Entzündung und Einwanderung von Granulozyten verbunden sind. Hierzu zählen die Psoriasis, atopische Dermatitis, Akne vulgaris und das entzündliche Stadium der Rosacea.

Die Rosacea ist eine Erkrankung unbekannter Ätiologie, die von manchen Autoren zum Formenkreis der Akne gezählt wird und sich auf dieser entwickeln und diese im zeitlichen Verlauf ablösen kann. Die Erkrankung wird in drei Stadien eingeteilt, von denen insbesondere die Rosacea II und III durch entzündliche Knötchen, Knoten und Plaques gekennzeichnet ist. Histologisch findet sich ein leukozytäres Infiltrat (Braun - Falco et al. (1995) Dermatologie und Venerologie. Heidelberg, Springer). Wegen der entzündlichen Komponente dieser Stadien mit Leukozyteninfiltration der Haut spiegelt das Crotonölmodell Aspekte dieser Erkrankung wider.

Bei der Behandlung der entzündlichen Form der Rosacea haben sich u.a. die zugelassenen Präparate Metronidazol (0.5 - 1.0 % in verschiedenen Vehikeln) und Azelainsäure (20 % in verschiedenen Vehikeln) bewährt (Rebora (2002) The management of rosacea. Am J. Clin Dermatol Vol 3(7), pp 489 - 498). Die Wirkung einer kombinatorischen Behandlung von Azelainsäure und Metronidazol wurde bisher nicht in vivo untersucht, es liegen lediglich Daten zum Vergleich der Wirksamkeit der Einzelsubstanzen bei Rosacea vor (Maddin (1999) A comparison of topical azelaic acid 20% cream and topical metronidazole 0.75% cream in the treatment of patients with papulopustular rosacea. Journal of the American Academy of Dermatology Vol 40(6): pp 961 - 965). Außerdem sind keine Untersuchungsergebnisse publiziert, die eine additive oder synergistische Wirkung einer Kombinationstherapie erwarten ließen.

Erstaunlicher Weise wurde gefunden, dass eine Kombinationstherapie von Metronidazol (0.75%) und Azelainsäure (20%) in Ethanol/Isopropylmyristat im Vergleich zum Vehikel signifikant therapeutisch effektiv waren, wohingegen die beiden Einzelsubstanzen Metronidazol (0.75%) bzw. Azelainsäure (20%) keine so hohe, therapeutische Effekte im Vergleich zum Vehikel zeigten. Die Wirksamkeit beinhaltete u.a. die Einwanderung von Leukozyten gemessen als Peroxidase-Aktivität in der Haut sowie die Einwanderung von neutrophilen Granulozyten gemessen als Elastase-Aktivität in der Haut (Tabelle 1).

Die Einwanderung dieser Zellpopulation in die Haut und Freisetzung proinflammatorischer Substanzen ist in die Pathogenese einer Reihe entzündlicher Erkrankungen involviert. Diese Erkrankungen beinhalten u.a. Rosacea, Akne, allergische und irritative Kontaktdermatitiden, atopische Dermatitis und Psoriasis Braun - Falco et al. (1995) Dermatologie und Venerologie. Heidelberg, Springer.

Die Leukozyteninfiltration in die durch Crotonöl entzündete Maushaut wird durch die kombinierte Behandlung mit Azelainsäure und Metronidazol im Vergleich zu Vehikelbehandlung signifikant inhibiert, wohingegen die einzelnen Wirkstoffe nicht so effektiv waren.

**Tabelle 1:**

| | | **Inhibition [%] der Hautinfiltration * von** | |
|---|---|---|---|
| **Auslösung** | **Behandlung** | **Neutrophilen** | **Granulozyten & Monozyten** |
| | | Elastaseaktivität | (Myelo-Peroxidaseaktivität) |
| Vehikel | Vehikel | 100 | 100 |
| Crotonöl | Vehikel | 0 | 0 |
| | (Negativkontrolle) | | |
| Crotonöl | Azelainsäure (20%) | 11 (n.s.) * | 21 (n.s.) * |
| | (Einzelsubstanz) | | |
| Crotonöl | Metronidazol (0.75%) | 18 (n.s.) * | 9 (n.s.) * |
| | (Einzelsubstanz) | | |
| Crotonöl | Azelainsäure (20%) + Metronidazol (0.75%) | 68 (p<0.05) * | 46 (p<0.05) * |
| | (erfindungsgemäße Zusammensetzung) | | |
| Crotonöl | Methylprednisolon | 95 (p<0.05) ^{*} | 90 (p<0.05) ^{*} |
| | (0.1%) | | |
| | (Positivkontrolle) | | |

| | | | |
|---|---|---|---|
| *Hemmung der Leukozyteninfiltration im Vergleich zu Crotonöl -Auslösung und Vehikelbehandlung (d.h. im Vergleich zur unbehandelten Positivkontrolle, wobei eine 100 %-ige Hemmung dem Wert der Vehikelauslösung und Vehikeltherapie entspricht, d.h. unbehandelte Negativkontrolle). | | | |

## Patentansprüche

1. Zusammensetzung, die die beiden folgenden Stoffe umfasst:
(i) mindestens eine Alpha,omega-n-alkandicarboxyl Säuren mit 7 bis 13 Kohlenstoffatomen oder deren Ester und
(ii) mindestens ein lmidazolderivat mit der allgemeinen Formel 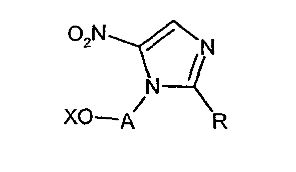
wobei
R ein Wasserstoffatom oder eine Alkylgruppe ist, die bis zu fünf Kohlenstoffatome enthält,
A eine gesättigte lineare Kette einer aliphatischen Hydrocarbongruppe ist, die zwei bis fünf Kohlenstoffatome enthält,
X ein Wasserstoffatom oder ein Akylrest einer monocarboxylischen oder dicarboxylischen aliphatischen oder aromatischen Säure ist.

2. Zusammensetzung nach Anspruch 1, wobei die Alpha,omega-n-alkandi-carboxyl Säuren Azelainsäure ist.

3. Zusammensetzung nach einem der vorherigen Ansprüche, wobei das Imidazolderivat ein 1-(2-hydroxyethyl)-2-methyl-5-nitroimidazol ist.

4. Zusammensetzung nach einem der vorherigen Ansprüche, wobei die Alpha,omega-n-alkandicarboxyl Säure oder deren Ester und
das Imidazolderivat
die folgenden pharmazeutischen Trägerstoffe umfasst:
Triacylglyceride, Propylenglycol, Polysorbate, Polyacrylsäure, Sojalecithin in einer wässrigen Phase, umfassend Wasser und Salze.

5. Zusammensetzung nach Anspruch 4, wobei die einzelnen Trägerstoffe unabhängig voneinander die folgenden Konzentrationen aufweisen:
Polyacrylsäure in einer Konzentration von 0,5 bis 2 Gewichtsprozenten,
Triacylglyceride in einer Konzentration von 0,5 bis 5 Gewichtsprozenten,
Propylenglycol in einer Konzentration von 5 bis 15 Gewichtsprozenten und
Polysorbate in einer Konzentration von 0,5 bis 3 Gewichtsprozenten.

6. Zusammensetzung nach einem der vorherigen Ansprüche als pharmazeutischer Wirkstoff.

7. Zusammensetzung nach Anspruch 6 zur Behandlung von Psoriasis, atopischer Dermatitis, Akne vulgaris und / oder Rosacea.

8. Zusammensetzung nach Anspruch 6 oder 7, wobei die Konzentrationen an Alpha,omega-n-alkandicarboxyl Säuren in dem Bereich 2 bis 40 % (Gewichtsprozente) liegt und
die Konzentration an lmidazolderivat in dem Bereich von mindestens 0,1 % bis 3 % (Gewichtsprozent) liegt.

9. Zusammensetzung nach einem der vorherigen Ansprüchen mit pharmazeutischen Zusätzen und/oder Trägersubstanzen

## Claims

1. A composition comprising the two following substances:
(i) at least one alpha,omega-n-alkane dicarboxylic acid with 7 to 13 carbon atoms or the esters thereof, and
(ii) at least one imidazole derivative with the general formula 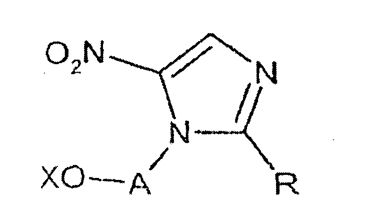
wherein
R is a hydrogen atom or an alkyl group, which comprises up to five carbon atoms,
A is a saturated linear chain of an aliphatic hydrocarbon group, which comprises two to five carbon atoms,
X is a hydrogen atom or an alkyl residue of a monocarboxylic or dicarboxylic aliphatic or aromatic acid.

2. The composition according to claim 1, wherein the alpha,omega-n-alkane dicarboxylic acid is azelainic acid.

3. The composition according to one of the preceding claims, wherein the imidazole derivative is a 1-(2-hydroxyethyl)-2-methyl-5-nitroimidazole.

4. The composition according to one of the preceding claims, wherein the alpha,omega-n-alkane dicarboxylic acid or the esters thereof and the imidazole derivative comprise the following pharmaceutical carrier substances:
triacylglyceride, propyleneglycol, polysorbate, polyacrylic acid, soy lecithin in an aqueous phase, comprising water and salts.

5. The composition according to claim 4, wherein the individual carrier substances independently from each other have the following concentrations:
polyacrylic acid in a concentration of 0.5 to 2 percent by weight,
triacylglyceride in a concentration of 0.5 to 5 percent by weight,
propyleneglycol in a concentration of 5 to 15 percent by weight, and
polysorbate in a concentration of 0.5 to 3 percent by weight.

6. The composition according to one of the preceding claims as a pharmaceutical effective substance.

7. The composition according to claim 6 for the treatment of psoriasis, atopic dermatitis, acne vulgaris and/or rosacea.

8. The composition according to claim 6 or 7, wherein the concentration of alpha,omega-n-alkane dicarboxylic acid is in the range from 2 to 40 % (percent by weight), and
the concentration of imidazole derivative is in the range from at least 0.1 % to 3 % (percent by weight).

9. The composition according to one of the preceding claims with pharmaceutical additives and/or carrier substances.

## Revendications

1. Composition comprenant les deux substances suivantes:
(i) au moins un acide alpha,oméga-n-alcane dicarboxylique avec 7 à 13 atomes de carbone ou les esters de celui-ci et
(ii) au moins un dérivé de l'imidazole ayant la formule générale 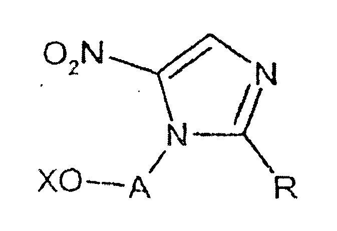
dans laquelle
R est un atome d'hydrogène ou un groupe alkyle, qui comprend jusqu'à cinq atomes de carbone,
A est une chaîne saturée linéaire d'un groupe hydrocarbure aliphatique, qui comprend deux à cinq atomes de carbone,
X un atome d'hydrogène ou un reste alkyle d'un acide monocarboxylique ou dicarboxylique aliphatique ou aromatique.

2. Composition selon la revendication 1, dans laquelle l'acide alpha,oméga-n-alcane dicarboxylique est l'acide azélaïnique.

3. Composition selon une des revendications précédentes, dans laquelle le dérivé de l'imidazole est un 1-(2-hydroxyéthyle)-2-méthyle-5-nitroimidazole.

4. Composition selon une des revendications précédentes, dans laquelle l'acide alpha,oméga-n-alcane dicarboxylique ou les esters de celui-ci et le dérivé de l'imidazole comprennent les substances porteuses pharmaceutiques suivantes:
le triacylglycéride, le propylèneglycol, le polysorbate, l'acide polyacrylique, la lécithine de soja dans une phase aqueuse, comprenant de l'eau et des sels.

5. Composition selon la revendication 4, dans laquelle les substances porteuses individuelles ont indépendamment l'une de l'autre les concentrations suivantes:
l'acide polyacrylique à une concentration de 0,5 à 2 pour cent en poids,
le triacylglycéride à une concentration de 0,5 à 5 pour cent en poids,
le propylèneglycol à une concentration de 5 à 15 pour cent en poids, et
le polysorbate à une concentration de 0,5 à 3 pour cent en poids.

6. Composition selon une des revendications précédentes comme substance active pharmaceutique.

7. Composition selon la revendication 6 pour le traitement du psoriasis, de la dermatite atopique, de l'acné vulgaire et/ou rosacée.

8. Composition selon la revendication 6 ou 7, dans laquelle la concentration de l'acide alpha,oméga-n-alcane dicarboxylique est dans la fourchette allant de 2 à 40 % (pour cent en poids), et
la concentration du dérivé de l'imidazole est dans la fourchette allant d'au moins 0,1 % à 3 % (pour cent en poids).

9. Composition selon une des revendications précédentes avec des additifs et/ou des substances porteuses pharmaceutiques.
